(19) Europäisches Patentamt
European Patent Office
Office européen des brevets

(11) Veröffentlichungsnummer : **0 099 987**
**B1**

(12) # EUROPÄISCHE PATENTSCHRIFT

(45) Veröffentlichungstag der Patentschrift : **20.08.86**

(51) Int. Cl.⁴ : **A 61 K 7/06**

(21) Anmeldenummer : **83106158.5**

(22) Anmeldetag : **23.06.83**

(54) **Haarbehandlungspräparat.**

(30) Priorität : **01.07.82 DE 3224585**

(43) Veröffentlichungstag der Anmeldung : **08.02.84 Patentblatt 84/06**

(45) Bekanntmachung des Hinweises auf die Patenterteilung : **20.08.86 Patentblatt 86/34**

(84) Benannte Vertragsstaaten : **AT BE CH DE FR GB IT LI NL**

(56) Entgegenhaltungen :
**DE-A- 2 451 918**
**DE-A- 2 847 439**
**FR-A- 875 246**
**US-A- 4 166 039**
**US-A- 4 237 112**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Christophliemk, Peter, Dr.**
**Rudolf-Breitscheidstrasse 61**
**D-4000 Düsseldorf 13 (DE)**
Erfinder : **Busch, Peter, Dr.**
**Gottfried-August-Bürger-Strasse 10**
**D-4006 Erkrath (DE)**
Erfinder : **Fischer, Detlef**
**Vennhausen 5**
**D-4000 Düsseldorf (DE)**
Erfinder : **Giede, Karl**
**Schlehenweg 12**
**D-4010 Hilden (DE)**
Erfinder : **Schreiber, Gerald**
**Emil-Barth-Strasse 109**
**D-4000 Düsseldorf 19 (DE)**
Erfinder : **Thiele, Klaus**
**Rügenweg 5**
**D-4018 Langenfeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 099 987 B1**

Jouve, 18, rue St-Denis, 75001 Paris, France

**Beschreibung**

Die Erfindung betrifft Haarbehandlungspräparate auf der Basis von Schichtsilikaten und Tensiden.

An Haarbehandlungspräparate oder Haarbehandlungsmittel werden eine Reihe von Anforderungen gestellt. Neben den üblichen Anforderungen, nämlich der Verträglichkeit mit dem Haar und der Kopfhaut sowie dem Reinigungsvermögen für verschiedene Verunreinigungen einschl. des Fettens von Haaren, sind dies insbesondere die Verminderung der Spliss-Rate, die Erniedrigung der elektrostatischen Aufladung, das Konditionieren und Avivieren der Haare ohne belastend zu wirken sowie eine Verbesserung der Frisierbarkeit und insbesondere der Naßkämmbarkeit.

Die Splissbildung hängt stark davon ab, wie sehr sich Haare verheddern können und beim anschließenden Kämmen mechanisch verletzt werden. Angaben zur Spliss-Bildung finden sich in den Veröffentlichungen von D.W. Cannell-, « Split ends and their repair », Cosmetics and Toiletries *94,* 29 (März 1979) und J.A. Swift and A.C. Brown, JSCC 23, 695 (1972) und JSCC 26, 289-297 (1975).

Produkte, die entscheidend die Splißrate erniedrigen, wie z. B. quaternierte Ammoniumverbindungen, die mindestens eine längere hydrophobierende Kohlenwasserstoffkette aufweisen, wirken bei mehrmaliger Anwendung auf das Haar stark belastend.

Alle bekannten Avivagemittel, die aus anionentensidhaltigen Mischungen aufziehen (meist quaternierte Polymere bzw. Polykondensate), erhöhen die elektrostatische Aufladung von Haaren. Darüber hinaus vermindern stark avivierende Produkte die Reibung zwischen den Fasern und wirken daher frisierverschlechternd.

Quaternierte Ammoniumverbindungen, die die elektrostatische Aufladung vermindern, ziehen aus anionentensidhaltigen Mischungen nicht auf Haar auf und wirken außerdem haarbelastend.

Die folgenden Veröffentlichungen können beispielhaft für die Verbesserung der Naßkämmbarkeit durch quaternäre Ammoniumverbindungen genannt werden :

M. Breuer, G. X. Gikas und I. T. Smith « Physical chemistry of hair condition », Cosmetics & Toiletries *94,* 29 (April 1979) ; M. L. Garcia und J Diaz « Combability Measurements on Human Hair », JSCC 27, 379 (1976) ; J. M. Quack und A. K. Reng, « Quaternäre Ammoniumverbindungen in der Kosmetik », Parfümerie und Kosmetik *56,* 157 (1975).

Aus den oben genannten Literaturstellen geht hervor, daß es bereits eine Reihe sehr wirksamer Mittel zur Verbesserung der Naßkämmbarkeit gibt. Zu den wirksamsten Mitteln zählen Fette, Öle und/oder quaternierte Ammoniumverbindungen mit einer oder mehreren langen hydrophoben Kohlenwasserstoffketten. Dieses Wirksubstanzen, die aus wässriger Mischung gut auf Haar aufziehen (sog. Nachbehandlungsmittel) wirken aus tensidhaltigen Formulierungen nicht oder nur sehr schwach. Sie besitzen den Nachteil, Haare stark elektrostatisch aufzuladen.

Aus der DE-OS 2 451 918 sind kosmetische Präparate bekannt, die aus einem Montmorillonit-Ton als Wirkstoff, mindestens einem die physiologische Wirkung des Tons erleichternden Hilfsstoff und ggfls. weiteren Zusatzstoffen bestehen. Als Montmorillonit-Ton werden Hectorit, Stevensit oder vorzugsweise Ghassoulit genannt. Als Hilfsstoffe werden Netzmittel wie anionische Netzmittel, z. B. Natriumlaurylschwefelsäureester oder Kondensationsprodukte einer Fettsäure mit Methyltaurin bzw. Fettsäure oder ein essentielles Öl genannt. Als Zusatzstoffe werden Lösungsmittel, Wasser, Meersalz, verschiedene Öle, Teer, Schwefel, Formaldehyd und verschiedene Duftstoffe genannt. Die Beispiele betreffen Waschmittel für normales Haar, Masken für fette Haut mit Neigung zu Akne und Spezialseifen für fette Haut und Toilettenseife für normale Haut.

Die vorliegende Erfindung stellt sich die Aufgabe, ein Haarbehandlungspräparat zu schaffen, das die Splißrate mindert, die elektrostatische Aufladung der Haare erniedrigt, die Naßkämmbarkeit verbessert, konditioniert und aviviert ohne belastend zu wirken und unter Reinigung des Haares die Frisierbarkeit erhöht.

Gelöst wird diese Aufgabe durch die Zurverfügungstellung des erfindungsgemässen Haarbehandlungspräparates auf der Basis von Schichtsilikaten und Tensiden und sein Herstellungsverfahren.

Gegenstand der Erfindung ist ein Haarbehandlungspräparat auf der Basis von Schichtsilikaten und Tensiden, das dadurch gekennzeichnet ist, daß es Schichtsilikate enthält, die vorher mit 20 bis 40 Gew.-% (bezogen auf wasserfreies Schichtsilikat) Tensiden vom anionischen, nichtionischen und/oder zwitterionischen Typ beladen worden sind, wobei das Schichtsilikat eine Dreischichtstruktur aufweist, in Wasser quellbar ist, eine Ladungsdichte bezüglich (Si, $Al)_4O_{10}$-Struktureinheiten von 0,25 bis 0,5 negativen Ladungseinheiten aufweist und in der Octaederschicht überwiegend Aluminium und/oder Magnesium enthält.

Gegenstand der Erfindung ist ferner ein Verfahren zur Herstellung der vorstehend beschriebenen Haarbehandlungspräparate, bei denen Schichtsilikate der genannten Art zu 5 bis 10 Gew.-% in Wasser eingerührt und nach einigen Stunden Quelldauer zentrifugiert und abgetrennt werden, worauf sie mit den Tensiden der genannten Art bei Temperaturen von 40 bis 70 °C beladen werden, indem man die flüssigen Tenside der auf die angegebenen Temperaturen erwärmten Schichtsilikat-Aufschlämmung zugibt, ggfls. noch eine Weile rührt, den Feststoff abtrennt und danach mit üblichen Formulierungsbestandteilen zur Herstellung von Haarbehandlungspräparaten mischt.

Überraschenderweise zeigen die erfindungsgemässen Haarbehandlungspräparate, die die bestimmten, vorher mit bestimmten Tensiden beladenen Schichtsilikate enthalten, die oben geschilderten Nachteile der Haarbehandlungsmittel des Standes der Technik nicht sondern zeigen eine wesentliche Verbesserung der Naßkämmbarkeit, eine starke Verminderung der elektrostatischen Aufladung der Haare, eine Verringerung der Splißrate sowie eine bessere Frisierbarkeit und eine verbesserte Konditionierbarkeit und Avivierbarkeit.

Bisher gab es keine Haarbehandlungsmittel, die die genannten Wirkungen mit Hilfe eines einzigen Wirkstoffes erzielten und es ist völlig überraschend, daß ein auf nur einem Wirkstoff beruhendes Haarbehandlungspräparat die Kombination der erwünschten Effekte bewirkt.

Im Folgenden werden die erfindungsgemäss geeigneten Schichtsilikate und Tenside beschrieben.

In den Schichtsilikaten liegt das Silikat wie bezeichnet als Schicht vor. Jeder Silikat-Tetraeder $(SiO_4)^{4-}$ ist über Sauerstoffbrücken mit drei anderen Silikat-Tetraedern zweidimensional zu Blättern der allgemeinen Zusammensetzung $(Si_2O_5)_n^{2-}$ vernetzt, ist darüber hinaus jedoch bis auf wenige mineralische Ausnahmen jeweils noch über die vierte Sauerstoffbrücke mit einem octaedrisch koordinierten Metallatom verknüpft. Auch diese Octaeder, in denen die metallischen Zentralatome von je 6 Sauerstoff-Atomen (überwiegend aus Hydroxylgruppen) umgeben sind, bilden typische Schichten. Durch dieses Strukturprinzip können je eine Tetraeder- und Octaederschicht zu einer Doppelschicht angeordnet sein, vermögen die zwischen zwei Tetraederschichten liegenden Octaederschichten jedoch auch mit ersteren sog. Dreischichtpakete zu bilden. Zwischen diesen Zwei- bzw. Dreischichtpaketen liegen Wasser-Zwischenschichten. Mineralische Vertreter der beiden Strukturtypen werden entsprechend Zweischicht- bzw. Dreischichtminerale genannt.

Bei ausschließlichem Vorliegen beispielsweise von Aluminium-Zentralatomen in der Octaederschicht als reine « Hydrargillit-Schicht » würde sich eine idealisierte Bruttozusammensetzung des (wasserhaltigen) Schichtsilikats von « $Al_2(OH)_2(Si_4O_{10}) \cdot nH_2O$ » ergeben. Ein derartiges idealisiertes Schichtsilikat wäre für die Erfindung wegen seiner Ladungsverhältnisse jedoch ungeeignet.

Charakteristisch für die erfindungsgemäss eingesetzten Schichtsilikate ist, daß die Zentralatome in der Octaeder-Schicht isomorph substituiert sind, Aluminium$^{3+}$ beispielsweise durch $Mg^{2+}$, $Fe^{2+}$ und/oder andere, bevorzugt zweiwertige Metall-Ionen. Silicium in der Tetraederschicht ist in geringerem Umfang substituiert, nahezu ausschließlich durch Aluminium. Hieraus resultiert in Abhängigkeit von Art und Umfang der isomorphen Substitution eine Ladungsdifferenz. Die Oberflächen der Schichtsilikate sind somit negativ geladen. Diese Ladungsdifferenzen werden ausgeglichen durch Alkali- und/oder Erdalkali-Ionen ; diese befinden sich zwischen den Schichten, sind in der Wasserzwischenschicht solvatisiert, austauschbar und somit Träger des Kationenaustauschvermögens dieser Mineralklasse.

Die allgemeine Bruttozusammensetzung (quellungsfähiger) Dreischichtsilikate lässt sich daher formulieren als

$$\underbrace{(M^{z+})^{(x+y)+}_{(x+y)/z}}_{\text{austauschfähige Kationen}} \underbrace{(H_2O)_n}_{\text{Wasser in der Zwischenschicht}} [\underbrace{(Me^{2+}, Me^{3+})^{(6-x)+}_{2-3} (OH)_2(Si_{4-y}\tilde{Al}_y)O_{10}}_{\text{starres, negativ geladenes Schichtpaket}}]^{(x+y)-}$$

$M^{Z+}$ sind hierin die austauschfähigen Kationen der Wertigkeit z,

$Me^{2+}$ und $Me^{3+}$ die zwei- und dreiwertigen Kationen in der Octaederschicht ; die Werte für x und y sind für die resultierende Gesamtladung charakteristische Besetzungszahlen. Eine derartige Summenformel wird beispielsweise bei A. Weiss und G. Lagaly, Kolloid-Z. und Z. für Polymere, *216*, *217*, 365-361 (1967), S. 360, verwendet. Bei bestimmten Schichtsilikat-Typen kann die Hydroxylgruppe dabei durch Fluorid substituiert sein (Hectorit).

Die Silikatschichten sind nicht unbegrenzt groß ; die Schichtsilikate bilden überwiegend kleine Partikel, oft mit blättchenförmiger Struktur bei einem Durchmesser von allenfalls einigen Mikrometern. An den Kanten dieser Blätter treten positive und negative Ladungen auf, da die oben beschriebenen Sauerstoffbrücken gebrochen sind. Während die negativen Ladungen (durch die beweglichen Kationen) neutralisiert werden, bleiben die positiven Ladungen im Nahbereich bestehen und führen an den Kanten zu einer positiven Gesamtladung. Durch Kanten-/Flächenbindungen vermögen derartige Schichtsilikate Gerüststrukturen in Form von « Kartenhäusern » aufzubauen. Hierdurch lassen sich auch die besonderen rheologischen Eigenschaften gequollener Schichtsilikate erklären, nämlich die Verdickungs- und Thixotropierungswirkung.

Infolge der verschiedenen Anordnungsmöglichkeiten von Tetraeder- und Octaederschichten, unterschiedlicher Zentralatome in den Octaederschichten und insbesondere auch infolge der vielfältigen Substitutionsmöglichkeiten ist die Variationsbreite der Schichtsilikate außerordentlich groß. Die in der Erdrinde verbreiteten Schichtsilikate unterscheiden sich daher je nach Lagerstätte auch bei gleichem Mineraltyp noch erheblich.

Für den Einsatz in der vorliegenden Erfindung sind alle solche Dreischichtminerale geeignet, die definierte Ladungsdichten auf der Schichtoberfläche sowie Ladungsdifferenzen zwischen Schichtoberfläche und Abbruchkante aufweisen und die weiter unten aufgeführten Tensid-Typen adsorbtiv zu binden vermögen.

Als Maß für die Ladungsdichte der Schichtsilikate lässt sich eine Schichtladung (x + y + z) als Überschussladung einer $(Si, Al)_4O_{10}^-$ Baueinheit gemäss,

$$M^{n+}_{\frac{x+y+z}{n}}[(Me^{3+}, Me^{2+}, Me^{+})^{(6-x)+}_{2-3}((OH)_{2-z}O_z)\ (Si_{4-y}Al_y)O_{10}]^{(x+y+z)-}$$

definieren und aus der vollständigen chemischen Analyse oder nach anderen Methoden ermitteln.

$M^{n+}$ sind hierin die austauschfähigen Kationen der Wertigkeit n,

$Me^{3+}$, $Me^{2+}$ und $Me^{+}$ Kationen in der Octaederschicht ; x, y und z sind die für die resultierende Gesamtladung wichtigen Besetzungszahlen (vgl. hierzu G. Lagaly und A. Weiss, Kolloid-Z. u. Z. Polymere *237*, 266-273, (1969), S. 268).

Diese Schichtladung (x + y + z) beträgt in den für die erfindungsgemässen Haarbehandlungspräparaten verwendenden Schichtsilikaten etwa 0,25 bis 0,5 negative Ladungseinheiten pro $(Si, Al)_4O_{10}$-Struktureinheit. Dementsprechend sind für die erfindungsgemässen Anwendungen die Dreischichtsilikate der Montmorillonit-Gruppe und von diesen insbesondere solche mit hohem Aluminiumgehalt von 10-20 Gew.-% oder Magnesiumgehalt von 20 bis 30 Gew.-%, bezogen auf das trockene Schichtsilikat, besonders gut geeignet. Montmorillonit und Hectorit sind gängige und weit verbreitete Vertreter dieses Mineral- und Ladungstyps und daher wirtschaftlich einzusetzen.

Wie eingangs erwähnt und in den dort genannten Literaturstellen ausgeführt, lassen sich die beweglichen Kationen in den Wasserzwischenschichten auch durch quaternäre Ammonium-Ionen mit langen organischen Resten austauschen. In Abhängigkeit von den Schichtsilikattypen und dem Aufbau des Ammoniumions (insbesondere der Kettenlängenverteilung) tritt dann eine intramolekulare Quellung auf, wodurch das durch Beladung mit quaternären Ammoniumionen organophylierte Schichtsilikat in geeigneten Lösungsmitteln dann als Verdickungsmittel wirkt. Diese Produkte haben die genannten Nachteile.

Die für die erfindungsgemässen Haarbehandlungspräparate verwendeten beladenen Schichtsilikate sind jedoch von völlig anderem Typ. Sie werden nicht über Kationenaustausch erhalten, sondern durch adsorbtive Anlagerung spezieller Tenside an die genannten bestimmten Schichtsilikate. Dementsprechend weisen die für die erfindungsgemässen Haarbehandlungspräparate geeigneten Anlagerungsprodukte nicht typischerweise ein Quell- und Thixotropierungsvermögen auf.

Dies unterscheidet die für die erfindungsgemässen Haarbehandlungspräparate verwendeten tensidbeladenen Schichtsilikate ganz wesentlich von den bisherigen technischen Anwendungen von Schichtsilikaten, bei denen zumindest eine der folgenden Substanzeigenschaften-Kationenaustauschvermögen, Quellvermögen, Verdickungsvermögen, Thixotropierungsvermögenausgenutzt wird.

Erfindungsgemäß eignen sich zur Beladung der bestimmten Schichtsilikate Tenside vom anionischen, nichtionischen und/oder zwitterionischen Typ. Derartige Aniontenside sind z. B. die Alkali- und Ammoniumsalze von z. B. Alkylsulfaten mit 8-18 Kohlenstoffatomen, Alkylpolyglykolethersulfaten mit 8-18 Kohlenstoffatomen in der Alkylgruppe und 1-6 Glykolethergruppen im Molekül, Alkylphenolpolyglykolethersulfaten mit 8-12 Kohlenstoffatomen in der Alkylgruppe und 1-6 Glykolethergruppen im Molekül, Fettsäurealkylolamid- und Fettsäurealkylolamidpolyglykolether-sulfaten, sulfatierten Fettsäuremonoglyceriden, primären und sekundären Alkansulfonaten, Alken- und Hydroxyalkansulfonaten mit 10-20 Kohlenstoffatomen, Sulfobernsteinsäure-monoalkylestern mit 8-18 Kohlenstoffatomen in der Alkylgruppe oder Dialkylestern mit 6-10 Kohlenstoffatomen in der Alkylgruppe, α-Sulfofettsäureniedrigalkylestern, Fettsäuren, Alkylpolyglykolethercarbonsäuren mit 8-18 Kohlenstoffen im Alkylrest und 2-6 Polyglykolethergruppen im Molekül, Acylsarkosinen, Acyltauriden und Acylisethionaten mit 8-18 Kohlenstoffatomen in der Acylgruppe, primären und sekundären Alkylphosphaten und Alkylpolyglykoletherphosphaten mit 8-18 Kohlenstoffatomen in der Alkylgruppe und 1-10 Polyglykolethergruppen im Molekül.

Geeignete nichtionogene Tenside sind z. B. die Anlagerungsprodukte von 4-40, vorzugsweise 4-20 Mol Ethylenoxid an Fettalkohole, Fettsäuren und Fettamine mit 8-18 Kohlenstoffatomen, an Alkylphenole mit 8-12 Kohlenstoffatomen im Alkylrest, an Fettsäureamide und Fettsäurealkylolamide, Fettsäure-mono-oder -diglyceride und an Sorbitanfettsäureester. Weiterhin sind auch solche Anlagerungsprodukte geeignet, die neben dem Ethylenoxid auch 1-10 Mol Propylenoxid angelagert enthalten. Geeignet sind schließlich auch die wasserlöslichen, 20-250 Ethylenglykolethergruppen und 10-100 Propylenglykolethergruppen enthaltenden Anlagerungsprodukte von Ethylenoxid and Polypropylenglykole, Alkylendiaminpolypropylenglykol und Alkylpolypropylenglykol mit 1-10 Kohlenstoffatomen in der Alkylkette sowie Aminoxide und Sulfoxide mit 8-18 Kohlenstoffatomen im Molekül.

Geeignete zwitterionische Tenside sind z. B. Alkylbetaine, Alkylamidopropyl-betaine, Alkylimidazoliniumbetaine, Alkylaminocarbonsäuren mit jeweils 8-18 Kohlenstoffatomen in der Alkylgruppe. Beispiele für solche zwitterionischen Tenside sind z. B. Kokosalkyldimethylaminoessigsäure, Kokosalkylamidopropyl-dimethylaminoessigsäure oder N-Hydroxyethyl-N-kokosalkylamidoethyl-glycin.

Bei der Herstellung der in den erfindungsgemässen Haarbehandlungspräparaten enthaltenen tensidbeladenen Dreischichtsilikaten kann wie folgt vorgegangen werden :

Als preiswerte Dreischichtsilikate können marktgängige Aktivbentonite verwendet werden ; diese

enthalten erhebliche Mengen Montmorillonit sowie unterschiedliche Mengen an Begleitmineralien. Je nach Aktivbentonit-Qualität müssen diese Begleitmineralien — insbesondere wenn es sich dabei um nicht quellfähige Minerale wie beispielsweise Quarz oder Feldspat handelt — weitgehend entfernt werden, da sie anwendungstechnisch stören. Die unerwünschten Begleitmineralien müssen bis auf einen Anteil von weniger als 5 %, vorzugsweise von weniger als 1 %, entfernt werden. Eine solche Abtrennung der verunreinigenden Stoffe läßt sich trocken über Windsichtung (gegebenenfalls sogar über Siebung) dann vornehmen, wenn diese Begleitminerale hohe Dichte oder große Partikeldurchmesser aufweisen. Technisch bevorzugt ist jedoch eine Separation der störenden Nebenbestandteile durch Zentrifugation einer Aufschlämmung. Hierzu wird das natürliche Schichtsilikat etwa zu 5-10 Gew.-% in Wasser eingerührt und nach einigen Stunden Quelldauer zentrifugiert. Durch Zentrifugation bei geeigneter Drehzahl (abhängig vom Zentrifugentyp) läßt sich eine nahezu ausschließlich gequollenes Schichtsilikat enthaltende Suspension erhalten ; die Mineralischen Nebenbestandteile bleiben zurück und werden verworfen. Diese zentrifugierte Suspension kann dann direkt zur Beladung mit Tensiden verwendet werden.

Diese Beladung kann prinzipiell zwar im gesamten Temperaturbereich vorgenommen werden, in dem sich die Komponenten handhaben lassen (ca. 0 bis 100 °C). Zweckmässig ist für die Beladung jedoch ein Temperaturbereich von 40 bis 70 °C, weil in diesem besonders homogene Produkte anfallen. Flüssige Tenside können als solche der vorgelegten Schichtsilikataufschlämmung zugefügt werden, pastöse oder gar feste werden — falls sie nicht in Wasser sehr gut verteilbar sind — zweckmässigerweise zunächst mit einer geringen Menge Lösungsmittel (beispielsweise Ethanol oder Isopropanol) gelöst oder zumindest angelöst.

Die auf 40 bis 70 °C aufgeheizte Schichtsilikat-Aufschlämmung wird in einem offenen Kessel vorgelegt, die flüssige, ggfls. erwärmte Tensidkomponente wird innerhalb weniger Minuten zugefügt. Zur Homogenisierung des Reaktionsprodukts wird noch ca. 30 Minuten unter Beibehaltung der Reaktionstemperatur weiter gerührt, der Feststoff abgetrennt und dann entweder direkt eingearbeitet oder aber zunächst weiter aufgearbeitet.

Die Abtrennung des festen Beladungsprodukts kann über Zentrifugation, einfacher jedoch meist über Filtration, vorgenommen werden. Das Beladungsprodukt wird (beispielsweise auf der Filternutsche) mit Wasser gewaschen und kann als Feuchtprodukt in dieser Form direkt eingesetzt werden. Es ist auch möglich, eine sich anschließende Trocknung, Zerkleinerung und/oder Sortierung der Partikel nach Dichte oder Größe durch Windsichtung bzw. Siebung durchzuführen.

Die optimale Menge Tensid bezogen auf Schichtsilikat in der Aufschlämmung ist in starkem Maße vom Schichtsilikat-Typ und Tensid-Typ abhängig. Keinesfalls sollte mehr Tensid zugefügt werden als vom Schichtsilikat sorbiert wird (außer wenn es sich um eine in der Gesamtrezeptur nicht störende Komponente handelt) ; das Austragen des überschüssigen Tensids ist dann mit zusätzlichem Aufwand verbunden. Bei zu niedrigem Tensidzusatz wird dagegen « Kapazität » verschenkt. Zweckmässigerweise bleibt man also mit der zugefügten Tensidmenge knapp unterhalb des Adsorptionsmaximums, welches durch Einzelversuche rasch zu bestimmen ist. Bei den gängigen und für die Erfindung infrage kommenden Tensiden sind dies Mengen von etwa 20 bis 40 Gew.-% Tensid, bezogen auf wasserfreies Schichtsilikat.

Die erhaltenen erfindungsgemäss beladenen Schichtsilikate können als Pulver, also als 100 %ige Ware, Verwendung finden, oder mit unterschiedlichen Hilfs- bzw. Wirkstoffen, die allgemein bekannt sind, zur Herstellung von beispielsweise Shampoos gemischt werden.

Die erfindungsgemässen Haarbehandlungspräparate wirken reibungsvermindernd und erniedrigen die elektrostatische Aufladung der Haare. Damit zusammenhängend werden die Haare weniger leicht an den Spitzen gesplisst oder abrasiv geschädigt. Weiterhin werden die Naßkämmbarkeit sowie die Frisierbarkeit verbessert.

Ein wesentliches Merkmal der erfindungsgemässen Haarbehandlungspräparate ist darin zu sehen, daß sie im Gegensatz zum Stand der Technik bestimmte Schichtsilikate enthalten, die vorher oder zunächst mit bestimmten Tensiden beladen worden sind. Das heisst, daß erhebliche Unterschiede sowohl im chemischen Verhalten als auch bei der Anwendung auftreten, wo man einerseits erfindungsgemäss bestimmte Schichtsilikate zuerst mit bestimmten Tensiden belädt und dann daraus unter Mischen mit üblichen Bestandteilen ein Shampoo herstellt oder wenn man Schichtsilikate, Natriumlaurylschwefelsäureester, also ein Tensid, und übliche Bestandteile zusammen gleichzeitig zur Herstellung eines Shampoo verarbeitet.

Zwischen dem bloßen Zusammenrühren von Tensiden und unbehandelten Schichtsilikaten in ein Shampoo einerseits (Stand der Technik) und dem Einrühren eines erfindungsgemäss beladenen Produkts in ein Shampoo andererseits bestehen in chemischer sowie anwendungstechnischer Hinsicht Unterschiede, wie aus Folgendem hervorgeht.

Verglichen wurde ein erfindungsgemäss beladenes Produkt mit dem Produkt, das nach Beispiel 2 der DE-OS 24 51 918 erhalten wurde.

Separiert man die silikatischen (wasserunlöslichen) Anteile in beiden Fällen aus dem Shampoo, so unterscheiden sich die erhaltene Feststoffe schon chemisch erheblich voneinander. Das erfindungsgemäss beladene Produkt erhält man nach Abtrennung praktisch unverändert zurück ; es enthält unverändert Beladungsprodukt in einer Größenordnung von 20 bis 40 Gew.-% entsprechend der

nachfolgenden Tabelle. 2, d. h. einen Anteil an Kohlenstoff von 8 % und darüber.

Das in ein Shampoo direkt eingerührte unbeladene Schichtsilikat dagegen belädt sich zwar dabei mit den verschiedenen Shampoo-Bestandteilen, die hier quasi miteinander « konkurrieren », diese Beladung hat jedoch einen wesentlich geringeren Umfang als beim erfindungsgemässen beladenen Produkt. In Nacharbeitung des Beispiels 2 der DE-OS 24 51 918 (NaCl anstelle von Meerzalz) wurde bei Verwendung des synthetischen Hectorits als Schichtsilikat-Komponente und Texapon N25 bzw. Texapon NT als Tensidkomponente nur eine Beladung entsprechend 1,1 % C bzw. 4,1 % C erzielt, während gemäss Tabelle 2, lfd. Nummer 7 und 8 Beladungen von 15,0 bzw. 11,2 % C erzielt wurden. Daraus sind die chemischen Unterschiede ganz eindeutig zu sehen.

Darüber hinaus quellen die in den erfindungsgemässen Haarbehandlungspräparaten enthaltenen beladenen Schichtsilikate nicht mehr, was ein wichtiges Kriterium ist, und führen daher bei einer Einarbeitung in Shampoos nicht zu starker (Nach) Verdickung. Dies ist jedoch bei Einrühren von unbeladenen Schichtsilikaten bekanntlich der Fall. Ferner bewirken die bereits beladenen Produkte keine — abträgliche — Fixierung und damit Unwirksammachung von Parfumbestandteilen der Shampoos und führen nicht zu Unverträglichkeiten mit hydrophilen und/oder oleophilen Shampoo-Bestandteilen wie Emulgatoren und Hilfsstoffen.

Die Erfindung wird nachstehend durch die folgenden Beispiele, anwendungstechnischen Prüfungen und Formulierungsbeispiele weiter erläutert.

## Beispiele

Für die Beispiele wurden drei natürliche marktgängige Schichtsilikate unterschiedlicher Herkunft und Zusammensetzung sowie ein synthetisches Produkt verwendet ; die Schichtladung (x + y + z) dieser Silikate wurde nach A. Weiss und G. Lagaly, loc. cit. zu 0,3 bis 0,5 negativen Ladungseinheiten bestimmt

1) ein hectoritähnliches gereinigtes Magnesiumsilikat mineralischen Ursprungs der Firma Kronos-Titan mit der Bezeichnung « Bentone EW » (für Produkt 1 und 2, Tabelle 2) ;

2) ein feinvermahlener Aktivbentonit (mit Soda « aktivierter » natürlicher Montmorillonit) der Firma Erbslöh, der gemäss Firmenangaben neben 80 bis 85 % Montmorillonit als mineralische Verunreinigungen etwa 5 bis 7 % Quarz, 5 bis 10 % Glimmer und 3 % Limonit aufweist und sehr feinteilig ist (87 % < 2 µm), für Produkt 3 und 4, Tabelle 2) ;

3) ein Hectorit aus nordamerikanischer Grube (Firma Langer & Co.), der jedoch etwa zur Hälfte noch Calcit, Montmorillonit und andere Schichtsilikate aufweist für Produkt 5 und 6, Tabelle 2) ;

4) ein synthetischer Hectorit hoher chemischer Reinheit für Produkt 7 und 8, Tabelle 2).

Die chemische Zusammensetzung dieser Schichtsilikate wurde röntgenfluoreszenzanalytisch bestimmt (Fluorgehalt titriert) und ist in Tabelle 1 zusammengefasst.

Für die Beladung wurden Aufschlämmungen der natürlichen Schichtsilikate von jeweils 5 Gew.-% in entionisiertem kalten Wasser hergestellt und diese nach 6stündiger Quellzeit mit einer Filterzentrifuge (400 mm Durchmesser) mit 2700facher Erdbeschleunigung zentrifugiert. Nach erfolgter Separation der schweren Anteile enthielten die Aufschlämmungen 3,0 % Aktivsubstanz bei Aktivbentonit, 3,5 % Aktivsubstanz bei Bentone EW bzw. 2,4 % Aktivsubstanz bei Hectorit. Der Aktivsubstanzgehalt der Aufschlämmungen wurde anhand von Proben durch 12stündiges Trocknen im Vakuumtrockenschrank bei 120 °C ermittelt. Für die Beladungen wurden diese zentrifugierten Aufschlämmungen verwendet. Im Falle des synthetischen Hectorits brauchten keine Nebenbestandteile separiert zu werden. Zur Beladung wurden hierbei die 5 %igen wässrigen Aufschlämmungen direkt ohne vorherige Zentrifugierung verwendet.

Als Tenside wurden verwendet :

Texapon(R)N 25 : Fettalkohol ($C_{12/14}$)-polyglykolether (2EO)-sulfat, Natriumsalz, ca. 28 % waschaktive Substanz, flüssig (Produkte 1, 3, 5, 8, Tabelle 2)

Texapon(R)NT : Fettalkohol ($C_{12/14}$)-polyglykolether (2EO)-sulfat-Triethanolammoniumsalz, ca. 35 % waschaktive Substanz, flüssig (Produkte 2, 4, 6, 7, Tabelle 2).

### Herstellung der beladenen Produkte

Jeweils 1 kg der Schichtsilikat-Aufschlämmung wurde entsprechend ihres Aktivsubstanzgehalts mit gleicher Menge Tensid beladen, also mit 24 g Tensid bei natürlichem Hectorit, 35 g Tensid bei Bentone EW, 30 g Tensid bei Aktivbentonit bzw. 50 g Tensid bei synthetischem Hectorit. Das Reaktionsgemisch wurde 30 Minuten bei 60 °C gerührt, der Feststoff abfiltriert, mit jeweils ca. 3 l entionisiertem Wasser gewaschen und 24 Stunden im Umlufttrockenschrank bei 70 °C getrocknet. Die getrockneten Produkte wurden verrieben und mit einer Stiftmühle vermahlen. Die Glühverluste (nach 30 Minuten bei 800 °C) und die mikroanalytisch ermittelten Kohlenstoffgehalte der Produkte in Abhängigkeit von Schichtsilikat und Tensid können Tabelle 2 entnommen werden.

Die erfindungsgemässen Haarbehandlungspräparate wurden verschiedenen anwendungstechnischen Prüfungen insbesondere auf Naßkämmbarkeit, elektrostatisches Verhalten sowie Splißbeständigkeit unterworfen.

1. Naßkämmbarkeit :

Die Naßkämmbarkeit kann genau und reproduzierbar quantifiziert werden — vgl. dazu W. Newman, G. L. Cohen und C. Hayes, « A quantitative characterization of combing », J. Soc. Cosm. Chem. *24*, 773-782 (1973 ; M. L. Garcia und I Diaz, « Combability measurements on Human Hair », J. Soc.-Cosm. Chem., 27, 379-398 (1976) ; A. M. Schwartz und D. C. Knowles, « Frictional effects in human hair », J. Soc. Cosm. Chem. *14*, 455-463 (1963) ; P. Busch und K. Thiele, « Eigendynamische Effekte an Haaren », « Beiträge zur Methodik der Kämmbarkeit », Ärzt. Kosmetologie *9*, 305-310 (1979). Bei der Bestimmung der Naßkämmbarkeit wird die Kraft gemessen, die von einer Kammschikane benötigt wird, um eine Haarsträhne bestimmter Länge zu durchkämmen. Die erfindungsgemässen tensidbeladenen Schichtsilikate wirken stark naßkämmbarkeitsverbessernd und zeigen hohe Naßkämmbarkeitsverbesserungen von 50 % und mehr.

Die Prüfungen erfolgten an Haarsträhnen (dunkelbraun, jeweils 3 g) der Spezifikation European dark, Code No. 6 634, Firma Alkinco/Klugman, New York. Die Strähnen wurden 30 Minuten mit 5,5 %iger Wasserstoffperoxid-Lösung bei pH 9,4 blondiert, anschließend 30 Minuten mit 6 %iger Thioglykolsäure bei pH 9,0 kaltgewellt und dann mit Texapon(R) N25 gereinigt. Hiernach erfolgte die Behandlung mit den erfindungsgemässen Produkten, welche hier als Trockensubstanz (jeweils 0,4 g Produkt pro Strähne) in die feuchten Haarsträhnen (ca. 50 % Wasser) eingerieben wurde (Fingermassage). Nach 5 Minuten Einwirkzeit bei Raumtemperatur wurde mit klarem Leitungswasser (20 °C) gespült. Die Naßkämmbarkeit wurde an den handtuchgetrockneten Haaren mittels einer Zug-Dehnungsapparatur bestimmt ; Methodik und Instrumentarium sind beschrieben (Busch und Thiele, Ärztl. Kosmetologie *9*, 305-310 (1979)). Es werden bei dieser Prüfung Kraft/Weg-Werte ermittelt, die beim Durchkämmen einer Haarsträhne mit Hilfe eines Kammsystems anfallen. Die Zuggeschwindigkeit der Maschine wurde auf 100 mm/Minute eingestellt. Als Kämme dienten zwei im Abstand von 1,5 mm gegenständig angeordnete Staubkämme.

Blindwerte — also Prüfungen ohne Einreiben des erfindungsgemässen Produkts — erbringen Naßkämmbarkeitswerte von 10 000 ± 799 mN. Naßkämmbarkeitswerte in diesem Bereich werden auch gemessen, wenn die reinen Schichtsilikate oder die Tenside allein verwendet werden.

2. Elektrostatische Aufladung :

Zur Methode der Messung der elektrostatischen Aufladung von Haaren können die Angaben von G. Blankenburg, V. Heß und D. Teasdale, Parfümerie und Kosmetik *62*, 169 (1981) sowie A. C. Lunn und R. E. Evans, J. Soc. Cosm. Chem. *28*, 549 (1977) herangezogen werden.

Zur Charakterisierung des elektrostatischen Verhaltens wurden die Haarsträhnen getrocknet, in einem abgeschlossenen Prüfraum bei 20 °C und 15 % relativer Luftfeuchtigkeit mit einer wie oben beschriebenen Apparatur 50 mal gekämmt und das resultierende Potential mit einem Voltmeter gegen Erde gemessen. Die so ermittelten Werte sind in Tabelle 2 aufgeführt. Blindproben ohne Einwirkung erfindungsgemässer Produkte erbringen hierbei Werte von − 130 ± 3 Volt. Auch hier führt die Verwendung der nicht erfindungsgemäß beladenen Schichtsilikate 1-4 oder der Tenside allein zu Potentialen entsprechend den Blindwerten. Mit Produkt Nr. 3 (Beispiel 3 gemäß der Erfindung) wurde ein Potential von + 56 Volt, mit Produkt Nr. 4 (Beispiel 4 gemäß der Erfindung) von + 73 Volt gemessen.

3. Splißrate :

Nicht zuletzt die günstigen Naßkämmbarkeiten und geringen elektrostatischen Aufladungen, hervorgerufen durch die erfindungsgemässen Haarbehandlungspräparate, führen dazu, daß die abrasive Haarschädigung beim Kämmen und die dadurch hervorgerufene Splißbildung außerordentlich gering ist.

Die Splißrate wurde visuell (mikroskopisch) durch Abzählung bestimmt. Die Bestimmungen erfolgten im Anschluß an 3 000 Kämmungen mit der beschriebenen Kämmapparatur bei 40 % relativer Luftfeuchtigkeit. In Blindproben nach Anwendung der unbeladenen Schichtsilikate oder der schichtsilikatfreien Tenside wurden Splißwerte von 17 bis 20 % erhalten. Mit Produkt Nr. 3 (Tabelle 2) wurde eine Splißrate von 3,3 %, mit Produkt Nr. 4 (Tabelle 2) von nur 1,7 % erzielt.

(Siehe Tabelle I Seite 8 f.)

Tabelle 1

Chemische Zusammensetzung der verwendeten Schichtsilikate (in %)

| Bestandteil | Aktivbentonit | Bentone EW | natürlicher Hectorit | synthetischer Hectorit |
|---|---|---|---|---|
| $SiO_2$ | 49.1 | 53.2 | 30.5 | 50.8 |
| $Al_2O_3$ | 17.9 | 0.8 | 1.2 | -- |
| MgO | 3.6 | 25.1 | 13.8 | 25.2 |
| $Na_2O$ | 3.7 | 3.1 | 1.9 | 3.9 |
| $Li_2O$ | 0.2 | 0.8 | 0.5 | 1.2 |
| CaO | 1.9 | 1.7 | 22.9 | -- |
| $K_2O$ | 1.0 | 0.2 | 0.2 | -- |
| $Fe_2O_3$ | 4.9 | 0.3 | 0.9 | -- |
| F | - | 3.9 | 2.1 | 4.2 |
| Glühverlust bei 800 °C | 17.0 | 11.4 | 27.9 | 15.3 |

Tabelle 2

Produktzusammensetzung und Naßkämmbarkeit

| Produkte | Schichtsilikat-Komponente | Tensid-Komponente | % Glühverlust | % C | Naßkämmbarkeit [mN] |
|---|---|---|---|---|---|
| 1 | Bentone EW | Texapon® N 25 | 24.9 | 10.4 | 3440 |
| 2 | | Texapon® NT | 45.2 | 22.1 | 4260 |
| 3 | Aktivbentonit | Texapon® N 25 | 23.3 | 8.6 | 5120 |
| 4 | | Texapon® NT | 22.4 | 9.4 | 4920 |
| 5 | natürlicher Hectorit | Texapon® N 25 | 30.2 | 10.7 | 4900 |
| 6 | | Texapon® NT | 32.0 | 11.7 | 6080 |
| 7 | synthetischer Hectorit | Texapon® NT | 38.9 | 15.0 | 5640 |
| 8 | | Texapon® N 25 | 32.1 | 11.2 | 5980 |

Anwendungsbeispiele

1. Pulvershampoo

99,5 Gew.-% Produkt 1 (Tabelle 2)
0,5 Gew.-% Parfümöl

Die Herstellung erfolgte durch Aufdüsen des Parfümöls in einem Lödige-Mischer.

2. Pulvershampoo

50 Gew.-% Produkt 2
49,5 Gew.-% Reisstärke
0,5 Gew.-% Parfümöl

Die pulverförmigen Komponenten wurden in einem Lödige-Mischer vorgelegt und durchmischt. Während des Mischvorganges wurde das Parfümöl aufgedüst.

3. Creme-Shampoo

20 Gew.-% Produkt 1 (Tabelle 2)
30 Gew.-% Fettalkohol ($C_{12/14}$)-polyglykolether (2EO)-sulfat, Na-Salz 28 %ig (Texapon (R) N25)
2 Gew.-% Kokosfettsäure ($C_{12-18}$)-diethanolamid (Comperlan (R) KD)
5 Gew.-% Natriummyristat
0,5 Gew.-% Parfümöl
42,5 Gew.-% Wasser

Die Komponenten wurden in einem Rührgefäß mit einem Pentraulik-Rührwerk gründlich gemischt. Das Gemisch wurde danach 3 x über einem Laborwalzenstuhl (Hartporzellanwalzen) geführt und homogenisiert.
Das fertige Shampoo war von cremiger Konsistenz und zeigte eine Viskosität von 400 Pa·s (20 °C), gemessen mit einem Brookfield Rotationsviskosimeter bei 5 UpM.

4. Creme-Shampoo

20 Gew.-% Produkt 5 (Tabelle 2)
30 Gew.-% Fettalkohol ($C_{12/14}$)-polyglykolether (2EO)-sulfat, Na-Salz 28 %ig (Texapon (R) N25)
10 Gew.-% Kokosalkylamidopropyl-dimethylaminoessigsäure 30 %ig (Dehyton K)
5 Gew.-% Ethylenglykolstearat (30 %ige Dispersion in Alkylethersulfat)
1 Gew.-% 2-Octyldodecanol
1 Gew.-% Balsamöl
0,3 Gew.-% Parfümöl
32,7 Gew.-% Wasser

Die Herstellung erfolgte wie in Beispiel 3. Das fertige Shampoo war von cremiger Konsistenz und zeigte eine Viskosität von 340 Pa · s (20 °C), gemessen mit einem Brookfield-Rotationsviskosimete bei 5 UpM.

5. Flüssiges Shampoo

10 Gew.-% Produkt 7 (Tabelle 2)
50 Gew.-% Fettalkohol ($C_{12/14}$)-polyglykolether (2EO)-sulfat, Na-Salz 28 %ig (Texapon (R) N25)
2 Gew.-% Kokosfettsäurediethanolamid (Comperlan (R) KD)
0,5 Gew.-% Parfümöl
37,2 Gew.-% Wasser
0,3 Gew.-% Kochsalz

Die Komponenten werden miteinander unter Rühren vermischt. Das fertige Shampoo ist von schwach opakem Aussehen und weist eine Viskosität von 12 Pa · s (20 °C) auf, gemessen mit dem Höppler-Kugelfallviskosimeter.

**Patentansprüche**

1. Haarbehandlungspräparat auf der Basis von Schichtsilikaten und Tensiden, dadurch gekennzeichnet, daß es Schichtsilikate enthält, die vorher mit 20 bis 40 Gew.-%, bezogen auf wasserfreies Schichtsilikat, Tensiden vom anionischen, nichtionischen und/oder zwitterionischen Typ beladen worden sind, wobei das Schichtsilikat eine Dreischichtstruktur aufweist, in Wasser quellbar ist, eine Ladungsdichte bezüglich $(SiAl)_4O_{10}$-Struktureinheiten von 0,25 bis 0,5 negativen Ladungseinheiten aufweist und in der Octaederschicht überwiegend Aluminium und/oder Magnesium enthält.

2. Haarbehandlungspräparat nach Anspruch 1, dadurch gekennzeichnet, daß das Schichtsilikat aus der Gruppe Montmorillonit, Bentonit, Hectorit und Ghassoulit ausgewählt ist.

3. Haarbehandlungspräparat nach Ansprüchen 1 und 2, dadurch gekennzeichnet, daß das Tensid ein Alkanolaminlaurylethersulfat ist, das ggfls. mit Ethylenoxid modifiziert worden ist.

4. Verfahren zur Herstellung von Haarbehandlungspräparaten nach Ansprüchen 1-3, dadurch gekennzeichnet, daß man ein ggfls. von Nebenbestandteilen befreites Schichtsilikat, wie in Anspruch 1 definiert, zu 5 bis 10 Gew.-% in Wasser einrührt, nach einigen Stunden Quelldauer zentrifugiert, abtrennt und anschließend bei 40 bis 70 °C mit einem flüssigen Tensid vom anionischen, nichtionischen und/oder zwitterionischen Typ belädt, wobei das Tensid in flüssiger Form zur Schichtsilikataufschlämmung gegeben, homogenisiert und dann der Feststoff abgetrennt und ggfls. mit üblichen Bestandteilen von Haarbehandlungsmitteln vermischt wird.

**Claims**

1. A hair treatment preparation based on layer silicates and surfactants, characterized in that it contains layer silicates which have been charged with from 20 to 40 % by weight, based on anhydrous layer silicate, of surfactants of the anionic, nonionic and/or zwitter-ionic type, the layer silicate having a three-layer structure, being swellable in water, having a charge density in regard to $(SiAl)_4O_{10}$ structural units of from 0.25 to 0.5 negative charge units and predominantly containing aluminium and/or magnesium in the octahedral layer.

2. A hair treatment preparation as claimed in Claim 1, characterized in that the layer silicate is selected from the group comprising montmorillonite, bentonite, hectorite and ghassoulite.

3. A hair treatment preparation as claimed in Claims 1 and 2, characterized in that the surfactant is an alkanolamine laurylether sulfate optionally modified with ethylene oxide.

4. A process for producing the hair treatment preparations claimed in Claims 1 to 3, characterized in that from 5 to 10 % by weight of a layer silicate of the type defined in Claim 1 optionally freed from secondary constituents is stirred into water, centrifuged after swelling for a few hours, separated off and then charged at 40 to 70 °C with a liquid surfactant of the anionic, nonionic and/or zwitterionic type, the surfactant being added in liquid form to the layer silicate suspension, homogenized and the solid subsequently separated off and optionally mixed with standard ingredients of hair treatment preparations.

**Revendications**

1. Préparation pour le traitement des cheveux à base de silicates et d'agents tensio-actifs, caractérisée en ce qu'elle contient des silicates, qui ont été chargés préalablement de 20 à 40 % en poids, calculé sur le phyllosilicate anhydre, d'agents tensio-actifs du type anionique, non ionique et/ou zwitterionique, ce silicate présentant une structure en trois couches, gonflant dans l'eau, présentant une densité de charge par rapport aux unités structurelles $(SiAl)_4O_{10}$ de 0,25 à 0,5 unités de charge négatives et contenant dans la couche octaédrique, principalement de l'aluminium et/ou du magnésium.

2. Préparation suivant la revendication 1, caractérisée en ce que le silicate est choisi dans le groupe formé par les montmorillonite, bentonite, hectorite et ghassoulite.

3. Préparation suivant l'une des revendications 1 et 2, caractérisée en ce que l'agent tensio-actif est un éthersulfate d'alcanolaminelauryle, qui a été éventuellement modifié avec de l'oxyde d'éthylène.

4. Procédé pour la fabrication de préparations pour le traitement des cheveux suivant les revendications 1 à 3, caractérisé en ce que l'on introduit en agitant un silicate d'où on a retiré les parties constituantes secondaires, tel qu'il est défini dans l'exemple 1, centrifuge au bout de quelques heures de durée de gonflement, sépare et ensuite, charge à 40 à 70 °C avec un agent tensio-actif anionique, non ionique et/ou du type zwitterionique, l'agent tensio-actif étant ajouté à l'empâtage de silicate sous la forme liquide, homogénéise, puis sépare la matière solide et mélange éventuellement avec les parties constituantes habituelles des agents pour le traitement des cheveux.